# EUROPEAN PATENT SPECIFICATION

(11) **EP 2 445 419 B1**
(45) Date of publication and mention of the grant of the patent: **12.02.2014**
(21) Application number: 10734557.1
(22) Date of filing: 24.06.2010
(51) Int. Cl.: A61B 17/17

(54) **A POSITIONING GUIDE AND A FEMUR BONE CUTTING GUIDE SYSTEM**
POSITIONIERUNGSFÜHRUNG UND SCHNEIDFÜHRUNGSSYSTEM FÜR OBERSCHENKELKNOCHEN
GUIDE DE POSITIONNEMENT ET SYSTÈME DE GUIDE DE DÉCOUPE D'OS FÉMORAL

(30) Priority: 24.06.2009 ZA 200904421
(43) Date of publication of application: 02.05.2012
(73) Proprietor: Custom Med Orthopaedics (Propietary) Limited, 7129 Western Cape (ZA)
(72) Inventor: HONIBALL, John, Robert, Stellenbosch, 7600 Cape Town (ZA)
(74) Representative: Gille Hrabal
(86) International application number: PCT/IB2010/052896
(87) International publication number: WO 2010/150222

(56) References cited:
- EP-A1- 1 669 033
- FR-A1- 2 819 168
- US-A- 5 853 415
- US-A1- 2008 161 815

## Description

### FIELD OF INVENTION

This invention relates to a positioning guide for use with a bone cutting guide assembly and to a femur bone cutting guide system for use in guiding the cutting of a patient's femur bone during a knee replacement surgical procedure. In this specification the terms knee replacement surgery and knee replacement surgical procedure shall be interpreted sufficiently broadly to include knee resurfacing and knee resurfacing surgical procedure, respectively.

### BACKGROUND TO THE INVENTION

US 5,853,415 discloses milling instrumentation comprising a femoral bracket consisting of a pair of base plates which are connectable to a patient's femur bone on either side of a lower extremity of the femur, and a milling guide which is connectable to the base plates, for guiding the cutting of the lower extremity of the patient's femur to provide for the fitment of a femoral component of a prosthetic knee joint. The milling instrumentation further includes an alignment guide and an Anterior-Posterior alignment guide (AP guide) which are removably connectable to the lower extremity of a patient's femur and which are together used by a surgeon to determine the location of the femoral bracket relative to the lower extremity of the patient's femur. The alignment guide uses a intramedullary canal of a patient as reference or datum which, it is assumed, approximates a longitudinal axis of the patient's femur. The alignment guide comprises an elongate intramedullary rod having a first end, an opposite second end, a longitudinal axis extending between the first and second ends; and a platform surrounding the rod near the second end thereof. The first end of the intramedullary rod is inserted through a hole drilled into a lower extremity of a femur and along a length of a shaft of the femur, and imbedded deeply into a intramedullary canal of the femur.

US Patent Application No. 12/039,849 published under US 2008/0161815 discloses an orthopaedic apparatus including a patient-specific alignment guide attachable to a surface of a bone being prepared for receiving a prosthetic implant. The guide includes at least first and second components and a coupling mechanism connecting the first and second components to one another. The first component has a first bone engagement surface and the second component has a second bone engagement surface, the first and second bone engagement surfaces anatomically matching corresponding first and second portions of the surface of the bone. At least one of the first and second components defines at least one alignment formation.

### SUMMARY OF INVENTION

According to a first aspect of the invention, there is provided a positioning guide for use with a bone cutting guide assembly for use in a knee replacement surgical procedure for guiding the cutting of at least one prosthetic joint locating face in an end region of a femur of a human patient, from which a portion of bone is to be removed, thereby to allow for the secure fitment of a prosthetic joint to the femur in a predetermined orientation which approximates the anatomical normality of the patient's knee joint, the bone cutting guide assembly including a bone cutting guide having at least one cutter guide formation for guiding a cutter for cutting said prosthetic joint locating face in said end region of the femur; and guide mounting means which can be fixedly secured to the femur and which includes mounting means to which the bone cutting guide is releasably mounted for releasably mounting the bone cutting guide relative to the femur,
the positioning guide including:
a bone mounting structure in the form of a moulding which is constructed from anatomical data obtained of said end region of the femur so as to define complementary locating formations which correspond to anatomical formations of said end region of the femur, thereby to provide for the secure fitment of the bone mounting structure to said end region of the femur; and
at least one attachment post which is fixed to and which projects outwardly from the bone mounting structure and to which the bone cutting guide of the bone cutting guide assembly is releasably mounted, in use, for positioning the bone cutting guide assembly as a unit, relative to the femur in an arrangement wherein the cutter guide formation of the bone cutting guide is located in a predetermined position relative to the femur so as to facilitate the cutting of said prosthetic joint locating face in the femur thereby to provide for the fitment of the prosthetic joint thereto.

The attachment post may have a releasable connecting formation for releasably connecting the attachment post to said bone cutting guide.

The positioning guide may include a pair of attachment posts.

According to a second aspect of the invention, there is provided a femur bone cutting guide system for use in a knee replacement surgical procedure for cutting at least one prosthetic joint locating face in an end region of a femur bone of a human patient, from which a portion of bone is to be removed, thereby to allow for the secure fitment of a prosthetic joint to the femur in a predetermined orientation which approximates the anatomical normality of the patient's knee joint, the bone cutting guide system including:
a bone cutting guide assembly including:
   a) a bone cutting guide having at least one cutter guide formation for guiding a cutter for cutting said prosthetic joint locating face in said end region of the femur; and
   b) guide mounting means which can be fixedly secured to the femur and which includes mounting means to which the bone cutting guide is releasably mounted for releasably mounting the bone cutting guide relative to the femur when the guide mounting means is secured thereto; and
a positioning guide as hereinabove described in accordance with the first aspect of the invention, for mounting the bone cutting guide assembly to the femur bone,
with the guide mounting means being fixedly secured to the femur bone after mounting of the bone cutting guide assembly to the attachment post, with the attachment post, the guide mounting means and the bone cutting guide being configured to permit separation of the bone cutting guide from the attachment post and from the guide mounting means to facilitate removal of the positioning guide, and remounting of the bone cutting guide to the guide mounting means after removal of the positioning guide.

The bone cutting guide may include attachment post mounting means for releasably mounting the bone cutting guide to the attachment post of the positioning guide.

The bone cutting guide may include complementary mounting means for releasably mounting the bone cutting guide to the mounting means of the guide mounting means.

The positioning guide may have a pair of attachment posts and the attachment post mounting means of the bone cutting guide may be in the form of a pair of complementary attachment post mounting formations.

The complementary mounting means of the bone cutting guide may be in the form of a pair of complementary mounting formations, each mounting formation being spaced towards opposite side regions of the cutting guide.

The guide mounting means of the bone cutting guide assembly may be in the form of a pair of side mounting plates, each side mounting plate having bone mounting means for releasably mounting the plate to a different opposite side region of the femur, in use.

The cutting guide may comprise a first cutting guide component and a second cutting guide component, the cutting guide components having complementary releasable securing formations for releasably securing the cutting guide components to one another.

### BRIEF DESCRIPTION OF THE DRAWINGS

Further features of the invention are described hereinafter by way of a non-limiting example of the invention, with reference to and as illustrated in the accompanying diagrammatic drawings. In the drawings:
Figure 1 shows a fragmentary perspective view of the bones of a human leg in their anatomically normal position;
Figure 2 shows a lower end view of the femur bone of the human leg shown in Figure 1;
Figure 3 shows a fragmentary perspective view of the femur of the human leg of Figure 1, which has been cut to fit a femoral component of a knee prosthesis thereto;
Figure 4 shows a fragmentary perspective view of the knee joint of Figure 1 to which a prior art knee prosthesis is connected;
Figure 5 shows a fragmentary sectional view of the prior art knee prosthesis of Figure 4, sectioned along section lines V - V of Figure 4;
Figure 6 shows a top view of a femoral sizing guide of a conventional prior art knee bone cutting guide system, the femoral sizing guide shown located against the femur of Figure 2;
Figure 7 shows a top view of an alignment guide rod of the conventional prior art knee cutting guide system of Figure 6, showing the alignment guide rod inserted into a hole drilled into the femur of Figure 6;
Figure 8 shows a fragmentary perspective view of a guide of the conventional prior art knee bone cutting guide system of Figure 6, showing the guide mounted on the alignment guide rod of Figure 7;
Figure 9 shows a fragmentary cross-sectional view of the femur bone of Figure 6, showing a mounting base of the conventional prior art knee cutting guide system of Figure 6 mounted onto the femur of Figure 6;
Figure 10 shows a fragmentary front view of a custom-made prior art knee-cutting guide;
Figure 11 shows a fragmentary side view of the custom-made prior art knee cutting guide of Figure 10 connected to a human femur and tibia bone;
Figure 12 shows a fragmentary side view of the custom-made prior art knee prosthesis, connected to the cut bone of Figure 11;
Figure 13 shows a fragmentary cross sectional view of a positioning guide of a bone cutting guide system in accordance with the invention, connected to the lower end region of a femur;
Figure 14 shows a perspective view of the bone cutting guide of the bone cutting guide system releasably connected to the positioning guide of Figure 13;
Figure 15 shows an exploded perspective view of the bone cutting guide system, disassembled, in accordance with the invention;
Figure 16 shows a perspective view of the bone cutting guide system of Figure 15, assembled;
Figure 17 shows a sectional side view of the bone cutting guide system of Figure 16, sectioned along section lines XVII - XVII of Figure 16;
Figure 18A shows a perspective view of a second embodiment of a femur bone cutting guide system in accordance with the invention;
Figure 18B shows a perspective view of the adaptor of the second embodiment of the femur bone cutting guide system shown in Figure 18A, mounted to the positioning guide thereof; and
Figure 18C shows a perspective view of the conventional prior art knee cutting guide system of Figure 6 mounted to the adaptor of Figure 18B.

### DETAILED DESCRIPTION OF THE DRAWINGS

The present invention relates to a femur bone cutting guide system for use in guiding the cutting of a patient's femur bone during a knee joint replacement surgical procedure.

The patient's knee joint may require replacement due to injury or deterioration caused by aging, or certain debilitating conditions, such as, for example, arthritis. An anatomically normal knee joint will be described below.

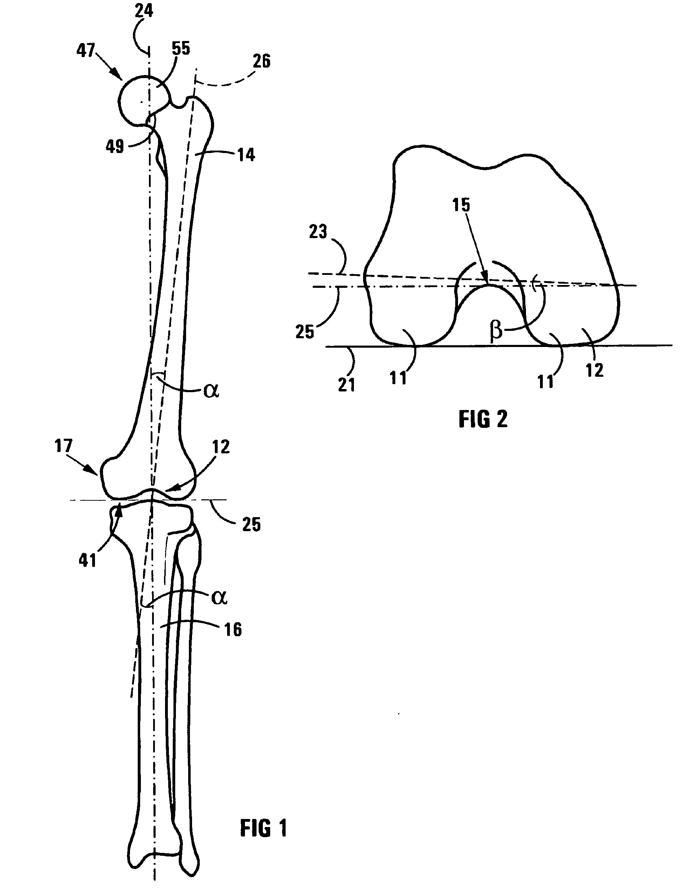

Figure 1 shows a perspective view of the bones of a human leg in their anatomically normal position. The bones include a femur 14 and a tibia 16, shown in their normal position defining an anatomically normal knee-joint 17.

The femur 14 has a lower extremity 12 at its distal end 41 and a femoral head 55 and a neck 49, at its proximal end 47. The femur 14 defines a longitudinal axis 26.

A mechanical axis 24 of the leg is also shown in Figure 1, the mechanical axis 24 extends from the centre of the head of the femur 55 and through the centres of the knee joint 17 and the ankle joint (not shown). In its anatomically normal position, the longitudinal axis 26 of the femur 14 is offset relative to the mechanical axis 24 by a deviation angle α of approximately 6º as shown in Figure 1. In its anatomically normal position, a longitudinal axis of the tibia 16 thus corresponds with the mechanical axis 24.

Figure 2 shows a lower end view of the lower extremity 12 of the femur 14 showing a transverse line 21 extending between the apices of anatomically normal posterior condyles 11. Figure 2 also shows a transverse axis 25 which is disposed parallel to the transverse line 21 and which extends through an apex of an intercondyloid fossa 15 of the lower extremity 12 of the femur 14. An external rotation axis 23 is angularly offset from the transverse axis 25 by an angle β of external rotation, the significance of which will be explained below.

Having described the normal anatomy of the knee 17, the cutting of the patient's joint bones during a typical knee joint replacement surgical procedure is described below.

During joint replacement surgery, at least one prosthetic joint locating face must be cut in an end region of the lower extremity of the femur by removing a portion of bone, so as to allow for the secure fitment of a prosthetic joint to the lower extremity of the femur in a predetermined orientation which approximates the anatomical normality of the patient's knee.

The cutting of the bones of the knee joint 17 includes the cutting of the lower extremity 12 of the femur 14 and the upper extremity of the tibia 16.

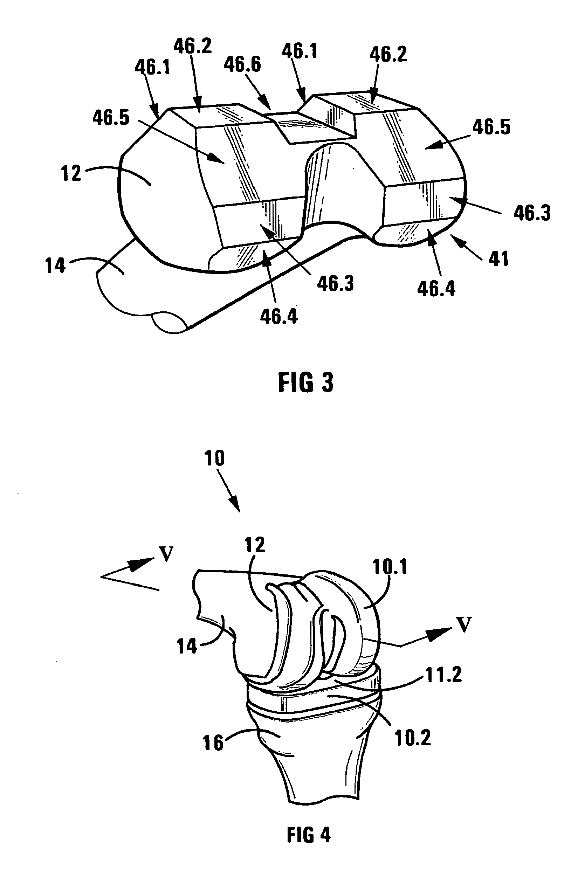

With reference to Figure 3 of the drawings, the cuts made to the lower extremity 12 of the femur 14 during a knee joint replacement surgical procedure, are shown. Figure 3 shows a lower extremity 12 which has been cut to form prosthetic joint locating faces 46 in an end region 41 of the lower extremity 12 of the femur 14. The cuts include an anterior cut 46.1, an anterior chamfer 46.2, a posterior chamfer 46.3, a posterior cut 46.4, a distal cut 46.5 and a notch 46.6. The orientation and position of the cuts 46 are critical to the fitment of the prosthesis, which must be fitted in a configuration which approximates the anatomical normality of the knee joint, as will be explained below.

With regards to the cutting of the tibia 16 of the knee joint 17, the tibia 16 is typically cut so as to define a flat face which extends perpendicularly with respect to the longitudinal axis of the tibia which coincides with the mechanical axis 24.

A number of different knee joint prostheses are supplied by different manufacturers, each manufacturer requiring different cutting configurations required to fit a particular prostheses.

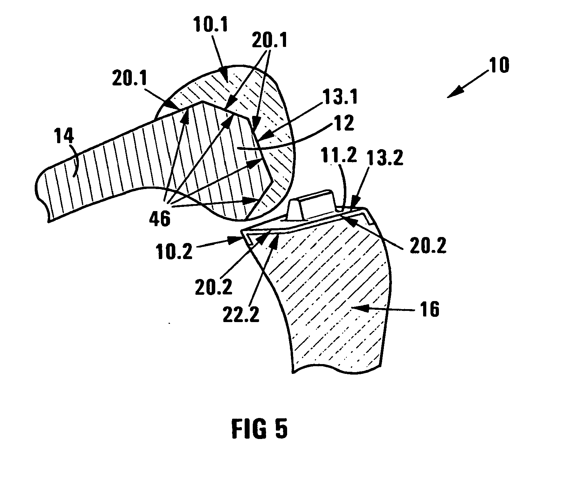

Referring to Figures 4 and 5, there is shown a typical conventional prior art knee prosthesis, which comprises two components, namely, a femoral component 10.1 and a tibial component 10.2.

Figures 4 and 5 show the lower extremity 12 of the femur 14 and the upper extremity of tibia 16 illustrating cut joint locating faces 46 and 22.2 of the femur 14 and tibia 16, respectively. The femoral component 10.1 includes a generally cup-shaped receiving formation 13.1 which defines a number of angled faces 20.1. The tibial component 10.2 includes a generally cup-shaped receiving formation 13.2 which defines a flat locating face 20.2. The cut locating faces 46 and 22.2 are securely located and cemented in the receiving formations 13.1 and 13.2, respectively, in a configuration which approximates the anatomical normality of the patient's knee joint 17.

To approximate anatomical normality of the patient's knee joint, the cut faces 46 are configured such that the prosthesis 10.1, once fitted, allows for the longitudinal axis of the tibia 16 to be angularly offset by an angle α of between 5º and 7º from the longitudinal axis 26 of the femur 14. The angle α provides for the alignment of the longitudinal axis of the tibia 16 with the mechanical axis 24 of the leg thereby to approximate the anatomical normality of the knee joint. Furthermore, the cut locating faces 46 are configured such that the femoral component 10.1, once fitted, allows for the angular rotation of the femoral component 10.1 by an angle β of external rotation of 3º relative to the transverse axis 25 as shown in Figure 2.

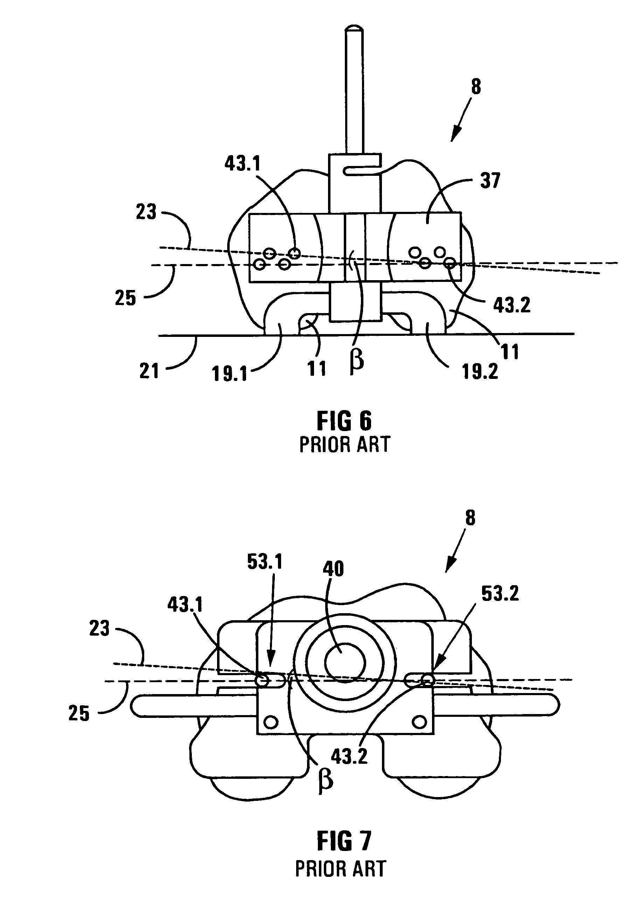

With reference to Figures 6, 7, 8 and 9, a conventional prior art knee cutting guide system 8, is shown. The prior art knee cutting guide system is used for cutting the joint locating faces 46 required for fitment of a particular prosthesis. Each prosthesis manufacturer provides a bone cutting guide system to facilitate cutting of the lower extremity 12 of the femur 14 in a particular configuration in order to fit the femoral component of the prosthesis to the patient. The conventional prior art cutting guide system includes a femoral sizing guide 37, a guide 34; a pair of mounting base plates 27, a mounting base 44; milling and cutting guides 45; and an alignment guide comprising a rod 40, the purpose of which will be described below.

In order to fit the knee prosthesis 10 to the patient, the patient is anesthetised and the skin and tissue covering the knee is cut and the knee joint is dislocated in order to expose the lower extremity 12 of the femur 14.

As a first step, with reference to Figure 6, the longitudinal axis 26 of the femur 14 is estimated by drilling hole 38 into the lower extremity 12 of the femur 14 and along the length of the shaft of the femur 14 thereby reaming the marrow cavity of the femur 14.

As a second step, with reference to Figure 7, guide arms 19.1 and 19.2, of the femoral sizing guide 37 are located against the posterior condyles 11 of the lower extremity 12 of the femur 14, so as to position hole drilling guides 43.1 and 43.2 relative to the lower extremity 12 of the femur 14 as shown in Figure 6. A pair of reference holes are then drilled into the lower extremity 12 of the femur 14 using hole drilling guides 43.1 and 43.2 to guide the drilling procedure.

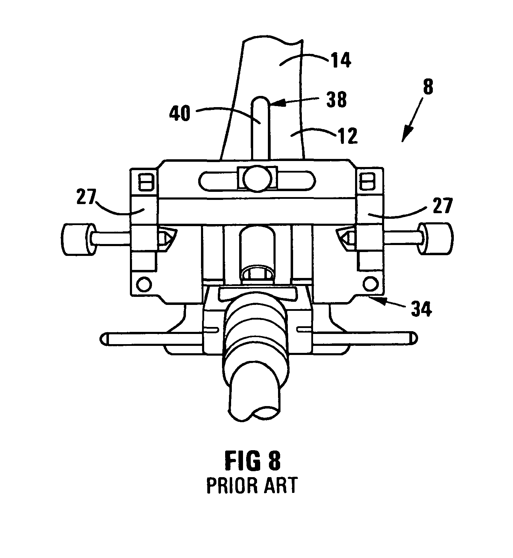

As a third step, the alignment guide rod 40 is then hammered into the hole 38, as shown in Figures 7 and 8. If correctly fitted, a longitudinal axis of the alignment guide rod 40 thus coincides with the longitudinal axis 26 of the femur 14. The alignment guide rod 40 includes plate defining alignment formations 53.1 and 53.2. The alignment guide rod 40 and plate are rotated until the drilled reference holes are aligned with the alignment formations 53.1 and 53.2, thereby to ensure that the alignment guide rod 40 is rotated relative to transverse axis 25, by an angle β of external rotation of 3°.

With reference to Figure 8, the alignment guide rod 40 is used to guide the location of the guide 34 relative to the bone. More particularly, the guide 34 is mounted onto the pair of mounting base plates 27 and the guide 34 is mounted onto the alignment guide rod 40. The guide 34 is then displaced relative to the alignment guide rod 40, to approximate the mechanical axis 24 of the femur 14. The mechanical axis 24 is estimated at an angle α of deviation of between 5 and 7º off-set from the approximated longitudinal axis 26, i.e. from the longitudinal axis of the alignment guide 40.

Once the guide 34 has been aligned to incorporate the abovementioned angles, the pair of mounting base plates 27 mounted onto the guide 34 are then connected to the femur 14 by means of pins (not shown). The guide 34 and alignment guide rod 40 are removed once the mounting base plates 27 have been secured to the patient's femur 14.

To remove the alignment guide rod 40 from the lower extremity 12 of the femur 14, the guide 34 is disconnected and removed from the mounting base plates 27 and thereafter the alignment guide rod 40 is removed from the femur 14. After removing the alignment guide rod 40, the milling and cutting guides 45 are connected to the base plates 27.

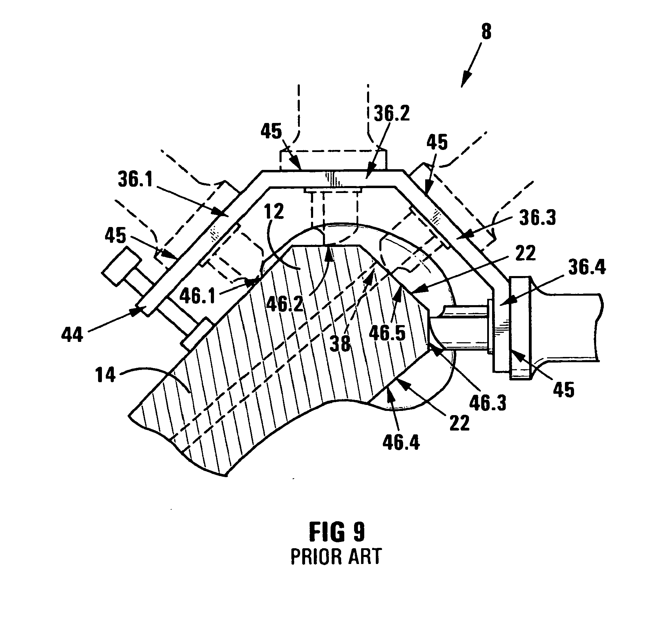

As shown in Figure 9, the milling and cutting guides 45, guide cutters for cutting of the lower extremity 12 of the femur 14. More particularly, the milling and cutting guides 45 provides guiding faces 36.1, 36.2, 36.3 and 36.4 for guiding the milling and cutting tools for cutting the joint locating faces 46 into the lower extremity 12.

Once the cuts have been affected, the milling and cutting guides 45 and mounting base 44 are removed from the mounting base plates 27 and the mounting base plates 27 are removed from the lower extremity 12 of the femur 14, by removal of the pins (not shown).

It will be appreciated that the success of the procedure is critically dependent upon the judgement and estimation of the surgeon, as the longitudinal axis 26 of the femur 14 is estimated and thereafter a mechanical axis 24 is determined relative to the estimated anatomical axis 26. As such, there is a need for a more precise manner of determining the correct location and configuration of the cuts 46 made to the lower extremity 12 of the femur 14. It will also be appreciated that the configuration of cutting guides will vary from one prosthesis manufacturer to another. Surgeons therefore become experienced in fitting prostheses from particular manufacturers. The prostheses 10 described thus far are commercially available in a variety of sizes to suit the size of the patient. The prosthesis 10 is in no way specific to a particular patient and is merely selected to be of a suitable size.

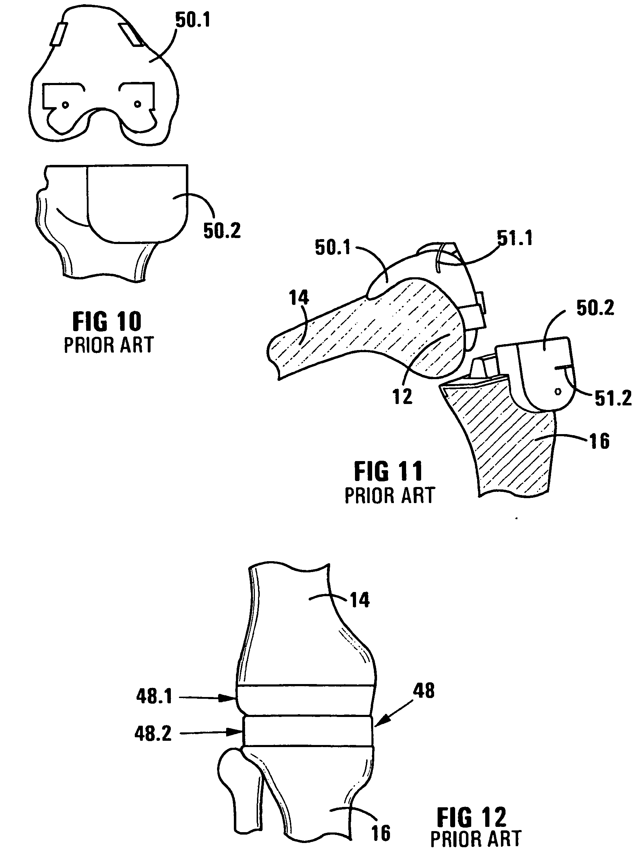

A more recent development in knee replacement surgery is a so-called patient-specific procedure, which, in some respects is an improvement of the procedure described above. Referring to Figure 10, 11 and 12, the patient-specific procedure involves the manufacture of a patient-specific knee replacement components of prosthesis 48.1 and 48.2, and an associated custom-made cutting guide system which includes cutting guide components 50.1 and 50.2. Each cutting guide component 50.1 and 50.2 includes receiving formations (not shown), which conform to the shape and configuration of a particular patient's knee joint. Each of the cutting guide components 50.1 and 50.2 define cutting guide formations 51.1 and 51.2, respectively.

The patient-specific procedure begins with a radiographic scan, which is performed to take precise measurements of a patient's knee. Computer software is then used to analyse the radiographic data and to build a 3-dimensional model of the patient's knee (not shown). Abnormalities in the knee caused by arthritis or other debilitating ailments, are taken into account, and digitally removed thereby to approximate the knee to its anatomical normality.

The computerised 3-D image of the prosthesis to be used in the patient's surgery is then shape matched to the anatomical model. This assists in determining the exact size and placement of the implant, based on the patient's own "normal" anatomy. Using the above information, the patient-specific prostheses 48.1 and 48.2 and corresponding custom-made bone cutting guides 50.1 and 50.2 are then manufactured specifically for the patient.

The custom made cutting guide components 50.1 and 50.2 have connecting formations (not shown) which correspond with the shape and configuration of the lower extremity 12 of the femur and of the upper extremity of the tibia 16, respectively, and which are attached to the ends of the lower extremity 12 and tibia 16 as shown in Figure 11.

The custom-made bone cutting guides 50.1 and 50.2 are fitted to the lower extremity 12 of the femur 14 and the upper extremity of the tibia 16, respectively, as shown in Figure 11. The cutting guide formations 51.1 and 51.2 are used for guiding cutters (not shown) used to cut the lower extremity 12 of the femur and the upper extremity of the tibia 16, respectively. More particularly, each cutting guide 50.1 and 50.2 guides the cutting of corresponding faces (not shown) which are cut into the extremities of the femur 14 and tibia 16, respectively. The cut faces correspond with and locate against corresponding faces (not shown), defined on the prostheses 48.1 and 48.2, respectively.

It will be appreciated that the success of the prior art patient-specific surgical procedure, described above, thus relies less on the skill and estimating abilities of the surgeon than is the case with the conventional prior art procedures described above.

A known disadvantage of the patient-specific knee prosthesis is that each prosthesis 48.1 and 48.2 and the bone cutting guides 50.1 and 50.2 have to be uniquely custom-made and cannot be tested and developed as extensively as the conventional prostheses 10.1 and 10.2. Furthermore, higher costs and longer production time is required for producing the patient-specific prosthetic knee device 48 and associated custom-made bone cutting guides 50.1 and 50.2.

A need has been identified for a device enabling a surgeon to use the conventional knee prostheses 10.1 and 10.2, together with a bone cutting guide configured to reliably locate the prostheses 10.1 and 10..2 in an optimum position which approximates the anatomical normality of the patient's knee joint 17 for a particular patient and without the need for estimation. Furthermore, a need exists for a reliable bone cutting guide system, which can be used, with a number of different prostheses supplied by different manufacturers of prostheses thereby allowing the surgeon a greater degree of choice in the selection of the most appropriate prosthesis.

Having described the prior art knee replacement prostheses and procedures above, various embodiments of a bone cutting guide system in accordance with the invention will now be described below.

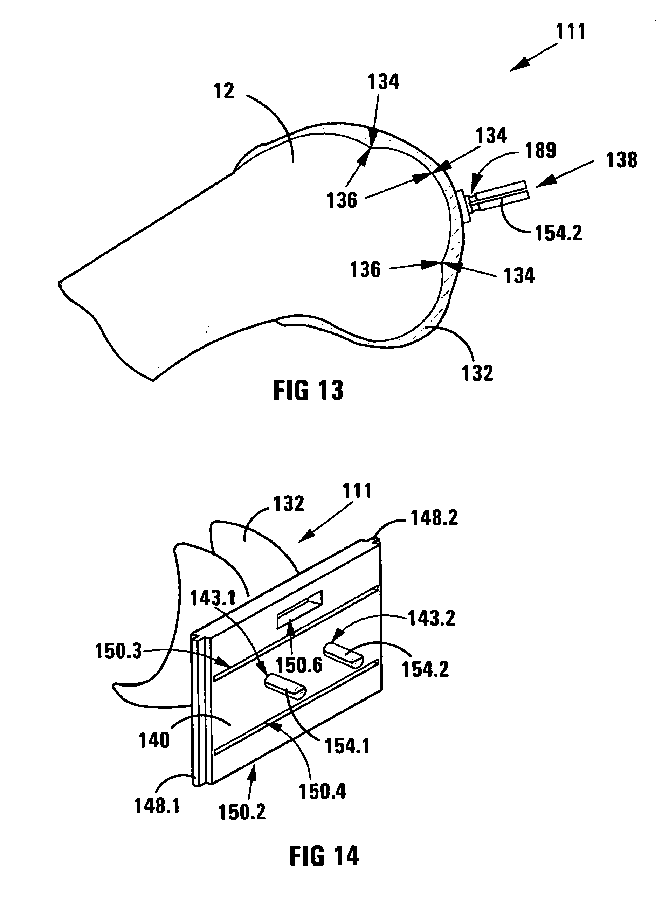

With reference to Figures 13 to 17 of the drawings, a femur bone cutting guide system, in accordance with the invention, is designated generally by the reference numeral 100. The bone cutting guide 100 is adapted for use in cutting prosthetic joint locating faces in a lower end region of a femur to facilitate the fitment of a prosthetic joint to the femur.

The femur bone cutting guide system 100 includes a bone cutting guide assembly 118 and a positioning guide 111 for mounting the bone cutting guide assembly 118 to the lower extremity 12 of the femur bone 14.

The bone cutting guide assembly 118 includes a bone cutting guide 124 and guide mounting means comprising a pair of side mounting plates 144.1, 144.2 defining holes 139 therethrough and securing pins 156 for securing the side mounting plates to the femur 14.

The bone cutting guide 124 defines a number of cutter guide formations for guiding a cutter 119 while cutting the prosthetic joint locating faces 46 in the lower extremity 12 of the femur 14. The bone cutting guide 124 includes a first cutting guide component 140 and a second cutting guide component 142.

The first cutting guide component 140 is in the form of a plate which defines attachment post mounting formations in the form of a pair of holes 143.1 and 143.2. The component 140 has releasable mounting formations defined on opposite side edges of the component 140 in the form of tongues 148.1 and 148.2. The cutter guide formations defined by the first cutting guide component 140 comprise an anterior abutment guide surface 150.1, a posterior abutment guide surface 150.2, a posterior chamfer slot 150.3, an anterior chamfer slot 150.4 and a notch slot 150.6.

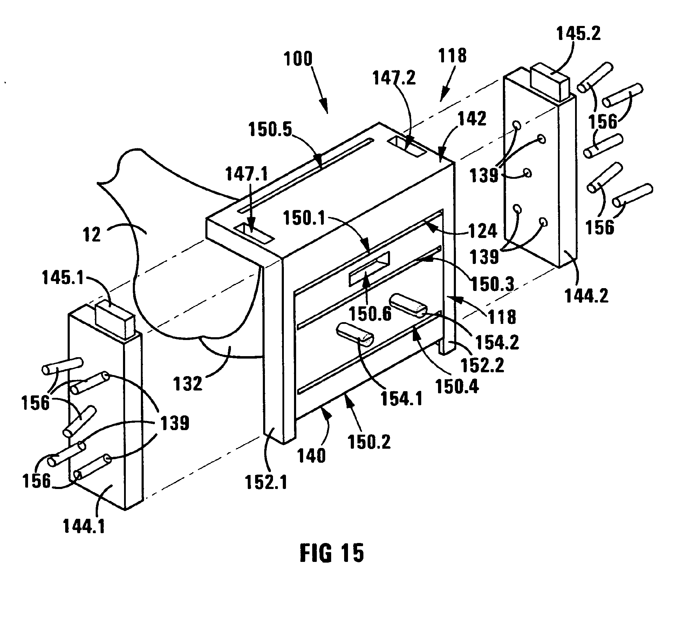

The second cutting guide component 142, with reference particularly to Figure 15, defines a pair of mounting formations in the form of a pair of slots 147.1 and 147.2 for releasably mounting the component 142 to the component 140. The component 142 defines a cutter guide formation in the form of a distal cutting slot 150.5. The second cutting guide component 142 further defines a pair of spaced arms 152.1 and 152.2 disposed at opposite sides thereof. Each arm 152.1 and 152.2 defines releasable mounting formations in the form of a groove (not shown), within which a different one of the corresponding tongues 148.1 and 148.2 of the first cutting guide component 140 are slidingly received when the first cutting guide component 140 and the second cutting guide component 142 are assembled.

The side mounting plates 144.1, 144.2 have mounting means in the form of mounting formations 145.1 and 145.2, respectively, which are received within the slots 147.1 and 147.2, respectively, of the component 142.

The positioning guide 111 comprises a bone mounting structure in the form of a moulding 132 and a pair of attachment posts 154.1, 154.2 which are fixed to and which project outwardly from the moulding 132 and to which the bone cutting guide 124 can be releasably mounted as will be explained below.

The moulding 132 is constructed from anatomical data obtained of the end region 41 of the lower extremity 12 of the femur 14 prior to surgery. The moulding 132 is thus constructed prior to the surgical procedure, from anatomical data obtained by means of a radiographic scan of the patient's lower extremity 12, from which scan, a three-dimensional model of the patient's lower extremity 12 is constructed (not shown). The moulding defines complementary locating formations 134 which correspond to anatomical formations 136 defined on the end region 41 of the lower extremity 12 of the femur 14. The locating formations 134 provide for secure fitment of the moulding 132 to the end regions 41 of the lower extremity 12 of the femur 14 in a specific position. More particularly, the locating formations 134 of the moulding 132 are configured to conform and correspond to the shape and configuration of the lower extremity of the femur. The moulding 132 is securely fitted, in use, onto the lower extremity 12 of the femur 14 with the complementary locating formations 134 of the moulding 132 corresponding with anatomical formations 136 defined on the end region 41 of the lower extremity 12 of the femur 14.

The attachment posts 154.1 and 154.2 have split ends and define connecting formations in the form of circumferential grooves 189, the purpose of which will be described below. The exact location and configuration of the attachment posts 154.1 and 154.2 is pre-determined when the moulding 132 is constructed.

The use of the positioning guide 111 and the bone cutting guide system 100 will now be explained with reference to Figures 13 to 17. With reference to Figure 13, the moulding 132 of the positioning guide 111 is fitted to the lower extremity of the femur as described above.

The locating formations 134 on the inner side of the moulding 132 correspond with the natural geometry of lower extremity 12. The position of the attachment posts 154.1 and 154.2 is thus fixed relative to the femur.

With reference to Figure 14, the first cutting guide component 140 is mounted to the moulding 132 by locating the attachment posts 154.1 and 154.2 within the holes 143.1 and 143.2 thereby to locate the first cutting guide component 140 with respect to the lower extremity 12 of the femur 14. The attachment posts 154.1 and 154.2 thus provide for location of the first cutting guide component 140 and thereby the second cutting guide component 142 of the cutting guide 124, when assembled to the component 140, in a pre-determined position relative to the lower extremity 12 of the femur 14 to be cut.

Figure 15 shows the second guide component 142 mounted to the first cutting guide component 140. The component 142 is releasably mounted to the component 140 by sliding the tongues 148.1 and 148.2 projecting from opposite sides of the component 140 into the grooves defined in the arms 152.1 and 152.2 of the component.

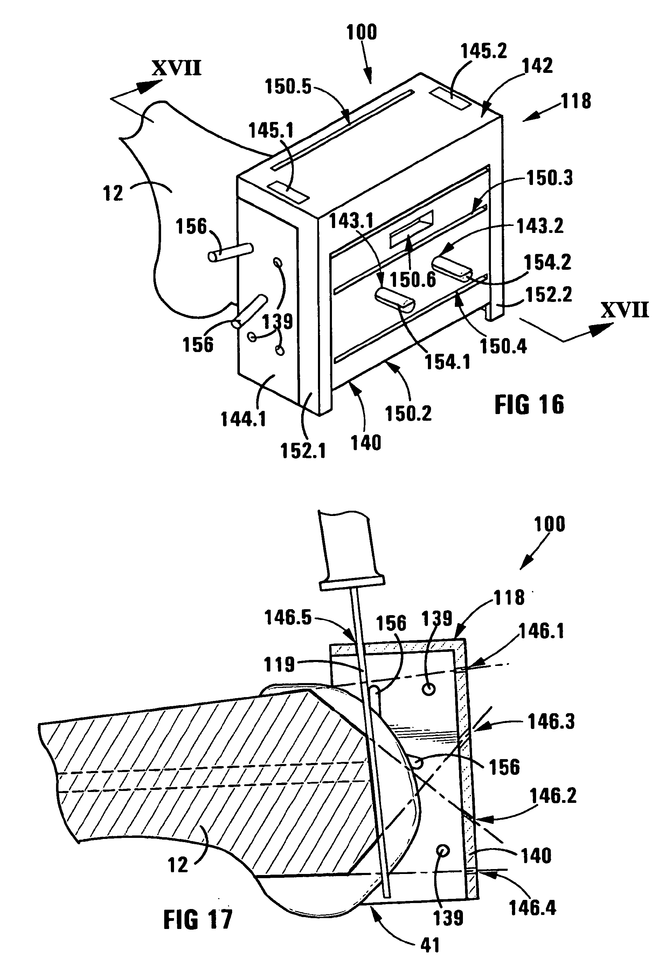

The side mounting plates 144.1, 144.2 are then connected to the component 142 by locating connecting formation 145.1 within slot 147.1 and by locating connecting formation 145.2 within slot 147.2 of the second cutting guide component 142. The side mounting plates 144.1, 144.2 are secured to opposite sides of the lower extremity 12 of the femur 14 by inserting the mounting pins 156 through the holes 139 and into the femur 14. Once the side mounting plates 144.1 and 144.2 are fixedly secured to the lower extremity 12 of the femur 14 by means of the mounting pins 156, the bone cutting guide assembly 118 is securely located relative to the lower extremity 12 of the femur 14 as illustrated in Figure 16.

The moulding 132 must be removed from its attachment to the patient's lower extremity 12 once the mounting plates 144.1, 144.2 have been secured to the femur 14, in order to facilitate cutting of the lower extremity 12 of the femur 14. In order to remove the moulding 132, the component 142 is separated from the guide component 140 by sliding the component 142 upwardly and away from the component 140. The component is then separated from the attachment posts of the moulding 132. The moulding 132 is then removed from the femur 14 leaving only the side mounting plates 144.1 and 144.2 fixed to the lower sides of the lower extremity 12 of the femur 14 by means of the mounting pins 156.

Once the moulding 132 has been removed, the component 140 is then remounted to the side mounting plates 144.1 and 144.2, and the component 142 is remounted to the component 140.

With reference to Figure 17, the components 140 and 142 are located in a predetermined spacial relationship with respect to the lower extremity 12 of the femur 14 providing for the accurate cutting of the lower extremity 12 using cutter 119. The cutter is guided by the anterior abutment guide surface 150.1, the posterior abutment guide surface 150.2, the posterior chamfer slot 150.3, the anterior chamfer slot 150.4, the distal cutting slot 150.5 and the notch slot 150.6.

The bone cutting guide assembly 118 is used to guide cutting devices 119 to cut the lower extremity 12 to produce the anterior cut 46.1, the posterior cut 46.4, the posterior chamfer 46.3, the anterior chamfer 46.2, the distal cut 46.5 and the notch 46.6 as shown in Figure 3.

It will also be appreciated that prior to the surgery, various measurements and calculations are performed in order to determine the optimal location and orientation of the prosthesis 10.1 for the specific patient's anatomy. The precise location and orientation of the attachment posts 154.1 and 154.2 determines the exact position of the bone cutting guide 124 and thereby the position and configuration of the cuts forming the joint locating faces 46 on the lower extremity 12 of the femur.

The invention extends to the bone mounting structure as defined and described hereinabove.

The invention also extends to the bone cutting guide 124 and to the bone cutting guide assembly 118 as defined and described hereinabove.

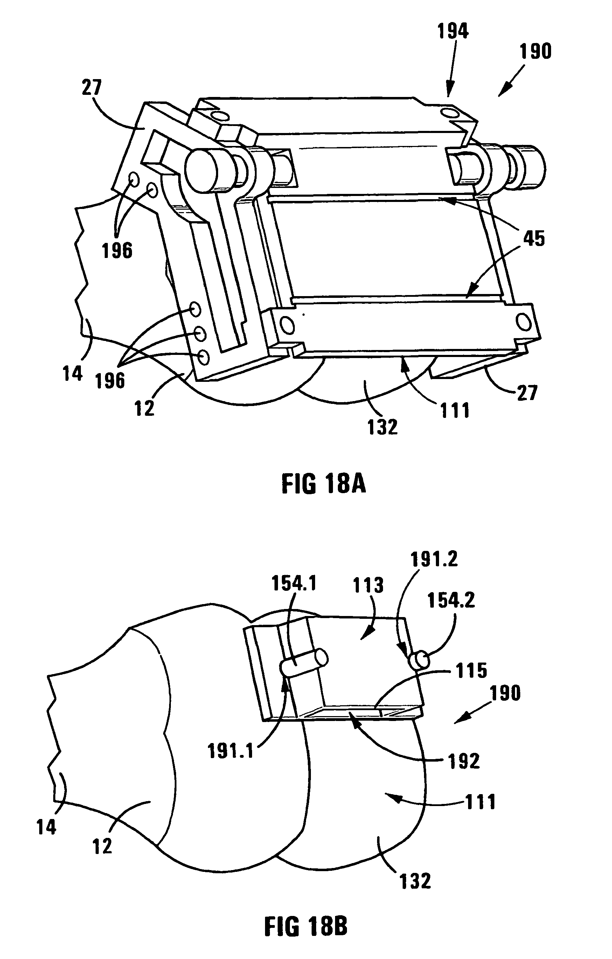

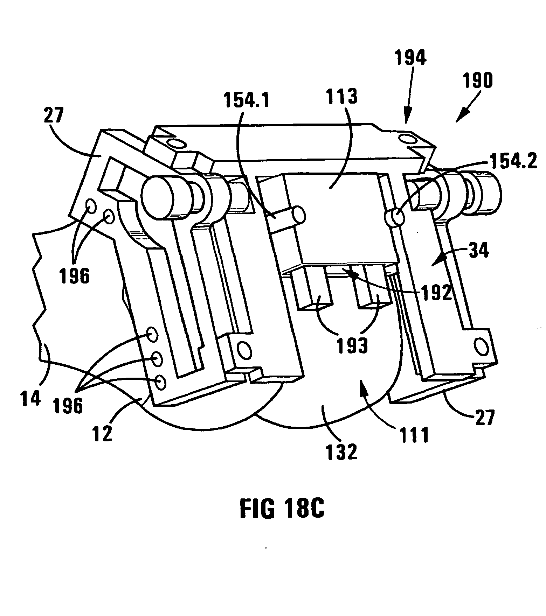

With reference to Figures 18A, 18B and 18C of the drawings, another embodiment of a femur bone cutting guide system, in accordance with the invention, is designated generally by the reference numeral 190.

The femur bone cutting guide system 190 includes a bone cutting guide assembly 194 and a positioning guide in the form of the positioning guide 111 of the femur bone cutting guide system 100, for mounting the bone cutting guide assembly 194 to the lower extremity 12 of the femur bone 14.

The bone cutting guide assembly 194 includes a bone cutting guide and guide mounting means comprising the pair of prior art mounting base plates 27 of the prior art knee cutting guide system 8 as illustrated in Figures 6, 7, 8 and 9. The prior art mounting base plates 27 are best shown in Figure 8.

The bone cutting guide includes a first cutting guide component in the form of an adaptor 113, a second cutting guide component in the form of the milling and cutting guides 45 of the prior art knee cutting guide system 8 and a third cutting guide component in the form of the guide 34 of the prior art knee cutting guide system 8.

The adaptor 113 defines a pair of apertures 191.1, 191.2 for releasably receiving the attachment posts 154.1, 154.2 of the positioning guide 111 therethrough; and defines a socket 192 for releasably receiving the connecting formations 193 of the prior art guide 34 therethrough.

The use of the femur bone cutting guide system 190 will be described below with reference to Figures 18A, 18B and 18C of the drawings. In use, the positioning guide 111 is mounted to the lower extremity 12 of the femur 14, in the same manner as is described above in relation to femur bone cutting guide system 100. The adaptor 113 is then mounted to the positioning guide 111 by receiving the attachment posts 154.1, 154.2 of the positioning guide 111 through the pair of apertures 191 of the adaptor 113. The conventional prior art guide 34 is then mounted to the adaptor 113 by receiving connecting formations 193 of the prior art guide 34 through the socket 192 of the adaptor 113. Once the conventional prior art guide 34 is correctly located relative to the lower extremity 12 of the femur 14, the pair of mounting base plates 27 of the conventional prior art knee cutting guide system are connected to the guide 34. After the base plates 27 are connected to the guide 34, the base plates 27 are fastened to the femur 14 by means of pins 196, as shown in the drawings. The guide 34 is then removed from its mounting to the base plates 27, in order to allow for the removal of the positioning guide 111 from its attachment to the lower extremity 12 of the femur 14. After the positioning guide 111 is removed, the guide 34 is re-mounted to the base plates 27 as shown in the drawings and the milling and cutting guides 45 are re-mounted to the guide 34, to commence cutting procedures.

It will be appreciated that the femur bone cutting guide system 190 provides a non-invasive manner of accurately locating the prior art guide 34 and the milling and cutting guides 45 of the prior art knee cutting guide system relative to the femur 14 bone. Furthermore, it will be appreciated that the bone cutting guide system 190 ameliorates the degree of estimation previously required to locate the guide 34 and the milling and cutting guides 45 of the prior art knee cutting guide system relative to the lower extremity 12 of the femur 14.

## Claims

1. A positioning guide (111) for use with a bone cutting guide assembly (118) for use in a knee replacement surgical procedure for guiding the cutting of at least one prosthetic joint locating face (46) in an end region (41) of a femur (14) of a human patient, from which a portion of bone is to be removed, thereby to allow for the secure fitment of a prosthetic joint to the femur in a predetermined orientation which approximates the anatomical normality of the patient's knee joint, the bone cutting guide assembly including a bone cutting guide (124) having at least one cutter guide formation (150.1, 150.2, 150.3, 150.4, 150.5, 150.6) for guiding a cutter (119) for cutting said prosthetic joint locating face in said end region of the femur; and guide mounting means which can be fixedly secured to the femur and which includes mounting means to which the bone cutting guide is releasably mounted for releasably mounting the bone cutting guide relative to the femur,
the positioning guide including:
a bone mounting structure; and
at least one attachment post (154.1, 154.2) which is fixed to and which projects outwardly from the bone mounting structure and to which the bone cutting guide of the bone cutting guide assembly is releasably mounted, in use, for positioning the bone cutting guide assembly as a unit, relative to the femur in an arrangement wherein the cutter guide formation of the bone cutting guide is located in a predetermined position relative to the femur so as to facilitate the cutting of said prosthetic joint locating face in the femur thereby to provide for the fitment of the prosthetic joint thereto,
**characterized in that** the bone mounting structure is in the form of a moulding (132) which is constructed from anatomical data obtained of said end region of the femur so as to define complementary locating formations (134) which correspond to anatomical formations (136) of said end region of the femur, thereby to provide for the secure fitment of the bone mounting structure to said end region of the femur.

2. The positioning guide as claimed in Claim 1, wherein the attachment post has a releasable connecting formation (189) for releasably connecting the attachment post to said bone cutting guide.

3. The positioning guide as claimed in Claim 1 or Claim 2, wherein the positioning guide includes a pair of attachment posts (154.1, 154.2).

4. A femur bone cutting guide system (100) for use in a knee replacement surgical procedure for cutting at least one prosthetic joint locating face in an end region of a femur bone of a human patient, from which a portion of bone is to be removed, thereby to allow for the secure fitment of a prosthetic joint to the femur in a predetermined orientation which approximates the anatomical normality of the patient's knee joint, the bone cutting guide system including:
a bone cutting guide assembly (118) including:
a) a bone cutting guide (124) having at least one cutter guide formation (150.1, 150.2, 150.3, 150.4, 150.5, 150.6) for guiding a cutter (119) for cutting said prosthetic joint locating face in said end region of the femur; and
b) guide mounting means which can be fixedly secured to the femur and which includes mounting means to which the bone cutting guide is releasably mounted for releasably mounting the bone cutting guide relative to the femur when the guide mounting means is secured thereto; and
the bone cutting guide system **characterized in that** the bone cutting guide system includes the positioning guide as claimed in Claim 1 for mounting the bone cutting guide assembly to the femur bone,
with the guide mounting means being fixedly secured to the femur bone after mounting of the bone cutting guide assembly to the attachment post, with the attachment post, the guide mounting means and the bone cutting guide being configured to permit separation of the bone cutting guide from the attachment post and from the guide mounting means to facilitate removal of the positioning guide, and remounting of the bone cutting guide to the guide mounting means after removal of the positioning guide.

5. The femur bone cutting guide system as claimed in Claim 4, wherein the bone cutting guide includes attachment post mounting means for releasably mounting the bone cutting guide to the attachment post of the positioning guide.

6. The femur bone cutting guide system as claimed in Claim 4 or Claim 5, wherein the bone cutting guide includes complementary mounting means for releasably mounting the bone cutting guide to the mounting means of the guide mounting means.

7. The femur bone cutting guide system as claimed in Claim 5, wherein the positioning guide has a pair of attachment posts (154.1, 154.2) and wherein the attachment post mounting means of the bone cutting guide is in the form of pair of complementary attachment post mounting formations (143.1, 143.2).

8. The femur bone cutting guide system as claimed in Claim 6, wherein the complementary mounting means of the bone cutting guide is in the form of a pair of complementary mounting formations (147.1, 147.2), each mounting formation being spaced towards opposite side regions of the bone cutting guide.

9. The femur bone cutting guide system as claimed in any one of Claims 4 to 8, wherein the guide mounting means of the bone cutting guide assembly is in the form of a pair of side mounting plates (144.1, 144.2), each side mounting plate having bone mounting means for releasably mounting the plate to a different opposite side region of the femur, in use.

10. The femur bone cutting guide system as claimed in any one of Claims 4 to 9, wherein the bone cutting guide comprises a first cutting guide component (140) and a second cutting guide component (142), the cutting guide components (140, 142) having complementary releasable securing formations (147.1, 147.2) for releasably securing the cutting guide components to one another.

## Patentansprüche

1. Positionierungsführung (111) zur Verwendung mit einer Knochenschneidführungsanordnung (118) für den Einsatz in einem Operationsprozess zum Austausch des Knies, um das Schneiden zumindest einer Anordnungsfläche (46) für eine Gelenkprothese in einem Endbereich (41) eines Oberschenkelknochens (14) eines menschlichen Patienten zu führen, von welchem ein Knochenabschnitt zu entfernen ist, um dadurch die sichere Anordnung einer Gelenkprothese an dem Oberschenkelknochen in einer vorbestimmten Ausrichtung zu ermöglichen, welche der anatomischen Normalität des Kniegelenks des Patienten nahe kommt, wobei die Knochenschneidführungsanordnung eine Knochenschneidführung (124) mit zumindest einer Schneidapparatführungsstruktur (150.1, 150.2, 150.3, 150.4, 150.5, 150.6) zum Führen eines Schneidapparates (119) zum Schneiden der Anordnungsfläche für die Gelenkprothese in dem Endbereich des Oberschenkelknochens aufweist; und eine Führungsmontagevorrichtung, welche an dem Oberschenkelknochen fest gesichert werden kann, und welche Montagevorrichtungen umfasst, an welchen die Knochenschneidführung lösbar angebracht ist, um die Knochenschneidführung bezüglich des Oberschenkelknochens lösbar anzubringen,
wobei die Positionierungsführung umfasst:
eine Knochenmontagestruktur; und
zumindest eine Befestigungsstütze (154.1, 154.2), welche an der Knochenmontagestruktur befestigt ist und von dieser nach außen vorspringt und an welcher die Knochenschneidführung der Knochenschneidführungsanordnung in Gebrauch lösbar angebracht ist, um die Knochenschneidführungsanordnung als eine Einheit gegenüber dem Oberschenkelknochen in einer Anordnung zu positionieren, in welcher die Schneidapparatführungsstruktur der Knochenschneidführung in einer vorbestimmten Position bezüglich des Oberschenkelknochens angeordnet ist, um das Schneiden der Anordnungsfläche für die Gelenkprothese in dem Oberschenkelknochen zu erleichtern und dadurch die Anordnung der Gelenkprothese daran zu ermöglichen,
**dadurch gekennzeichnet, dass** die Knochenmontagestruktur in Form eines Formteils (132) ausgebildet ist, welches anhand von anatomischen Daten, die von dem Endbereich des Oberschenkelknochens erhalten wurden, konstruiert ist, um komplementäre Anordnungsstrukturen (134), die den anatomischen Strukturen (136) des Endbereichs des Oberschenkelknochens entsprechen, zu definieren und dadurch die sichere Anordnung der Knochenmontagestruktur an dem Endbereich des Oberschenkelknochens zu gewährleisten.

2. Positionierungsführung gemäß Anspruch 1, wobei die Befestigungsstütze eine lösbare Verbindungsstruktur (189) aufweist zur lösbaren Verbindung der Befestigungsstütze mit der Knochenschneidführung.

3. Positionierungsführung gemäß Anspruch 1 oder Anspruch 2, wobei die Positionierungsführung ein Paar Befestigungsstützen (154.1, 154.2) aufweist.

4. Schneidführungssystem für Oberschenkelknochen (100) zum Einsatz in einem Operationsprozess zum Austausch des Knies zum Schneiden zumindest einer Anordnungsfläche für eine Gelenkprothese in einem Endbereich eines Oberschenkelknochens eines menschlichen Patienten, von welchem Oberschenkelknochen ein Knochenabschnitt zu entfernen ist, um dadurch die sichere Anordnung einer Gelenkprothese an dem Oberschenkelknochen in einer vorbestimmten Ausrichtung zu ermöglichen, welche der anatomischen Normalität des Kniegelenks des Patienten nahe kommt, wobei das Knochenschneidführungssystem aufweist:
eine Knochenschneidführungsanordnung (118), welche umfasst:
a) eine Knochenschneidführung (124) mit zumindest einer Schneidapparatführungsstruktur (150.1, 150.2, 150.3, 150.4, 150.5, 150.6) zum Führen eines Schneidapparates (119) zum Schneiden der Anordnungsfläche für die Gelenkprothese in dem Endbereich des Oberschenkelknochens; und
b) eine Führungsmontagevorrichtung, welche an dem Oberschenkelknochen fest gesichert werden kann, und welche Montagevorrichtungen umfasst, an welchen die Knochenschneidführung lösbar angebracht ist, um die Knochenschneidführung bezüglich des Oberschenkelknochens lösbar anzubringen, wenn die Führungsmontagevorrichtung daran befestigt ist; und
wobei das Knochenschneidführungssystem **dadurch gekennzeichnet ist, dass** das Knochenschneidführungssystem die Positionierungsführung gemäß Anspruch 1 zur Montage der Knochenschneidführungsanordnung an dem Oberschenkelknochen aufweist,
wobei die Führungsmontagevorrichtung nach der Montage der Knochenschneidführungsanordnung an der Befestigungsstütze an dem Oberschenkelknochen fest gesichert wird, wobei die Befestigungsstütze, die Führungsmontagevorrichtung und die Knochenschneidführung derart ausgebildet sind, dass sie eine Trennung der Knochenschneidführung von der Befestigungsstütze und von der Führungsmontagevorrichtung erlauben, um das Entfernen der Positionierungsführung und eine erneute Montage der Knochenschneidführung an der Führungsmontagevorrichtung nach dem Entfernen der Positionierungsführung zu vereinfachen.

5. Schneidführungssystem für Oberschenkelknochen gemäß Anspruch 4, wobei die Knochenschneidführung Montagevorrichtungen zur Montage an der Befestigungsstütze aufweist, um die Knochenschneidführung lösbar an der Befestigungsstütze der Positionierungsführung anzubringen.

6. Schneidführungssystem für Oberschenkelknochen gemäß Anspruch 4 oder Anspruch 5, wobei die Knochenschneidführung komplementäre Montagevorrichtungen aufweist zur lösbaren Anbringung der Knochenschneidführung an den Montagevorrichtungen der Führungsmontagevorrichtung.

7. Schneidführungssystem für Oberschenkelknochen gemäß Anspruch 5, wobei die Positionierungsführung ein Paar Befestigungsstützen (154.1, 154.2) aufweist und wobei die Montagevorrichtungen zur Montage an der Befestigungsstütze der Knochenschneidführung in Form eines Paars komplementärer Strukturen zur Montage an der Befestigungsstütze (143.1, 143.2) ausgebildet sind.

8. Schneidführungssystem für Oberschenkelknochen gemäß Anspruch 6, wobei die komplementären Montagevorrichtungen der Knochenschneidführung in Form eines Paares komplementärer Montagestrukturen (147.1, 147.2) ausgebildet sind, wobei jede Montagestruktur von gegenüberliegenden Seitenbereichen der Knochenschneidführung beabstandet angeordnet ist.

9. Schneidführungssystem für Oberschenkelknochen gemäß irgendeinem der Ansprüche 4 bis 8, wobei die Führungsmontagevorrichtung der Knochenschneidführungsanordnung in Form eines Paares von Seitenmontageplatten (144.1, 144.2) ausgebildet ist, wobei jede Seitenmontageplatte Knochenmontagevorrichtungen aufweist zur lösbaren Anbringung der Platte in Gebrauch an einem unterschiedlichen gegenüberliegenden Seitenbereich des Oberschenkelknochens.

10. Schneidführungssystem für Oberschenkelknochen gemäß irgendeinem der Ansprüche 4 bis 9, wobei die Knochenschneidführung eine erste Schneidführungskomponente (140) und eine zweite Schneidführungskomponente (142) aufweist, wobei die Schneidführungskomponenten (140, 142) komplementäre, lösbare Befestigungsstrukturen (147.1, 147.2) zur lösbaren Befestigung der Schneidführungskomponenten aneinander aufweisen.

## Revendications

1. Guide de positionnement (111) pour l'utilisation avec un ensemble de guide de découpe d'os (118) destiné à être utilisé dans une procédure d'opération de remplacement de genou pour guider le découpe d'au moins une surface de mise en place d'une prothèse articulaire dans une zone d'extrémité (41) d'un os fémoral (14) d'un patient humain, duquel os fémoral il faut enlever une partie d'os pour ainsi permettre d'attacher la prothèse articulaire de manière sûre à l'os fémoral dans une orientation prédéterminée qui est proche de la normalité anatomique de l'articulation du genou du patient, l'ensemble de guide de découpe d'os comprenant un guide de découpe d'os (124) ayant au moins une structure de guide de découpeur (150.1, 150.2, 150.3, 150.4, 150.5, 150.6) pour guider un découpeur (119) pour découper la dite surface de mis en place de la prothèse articulaire dans la dite zone d'extrémité de l'os fémoral ; et des moyens de montage de guide susceptible d'être fermement attachés à l'os fémoral, lesquels comprennent des moyens de montage sur lesquels le guide de découpe d'os est monté de manière amovible pour monter le guide de découpe d'os par rapport à l'os fémoral de manière amovible,
le guide de positionnement comprenant :
une structure de montage d'os ; et
au moins un support d'attache (154.1, 154.2) qui est fixé à la structure de montage d'os et fait saillie vers l'extérieur à partir de celle-ci et sur lequel l'ensemble de guide de découpe d'os est monté de manière amovible pendant l'usage pour positionner l'ensemble de guide de découpe d'os comme une unité par rapport à l'os fémoral dans une disposition dans laquelle la structure de guide de découpe du guide de découpe d'os est disposée dans une position prédéterminée par rapport à l'os fémoral pour faciliter le découpe de la surface de mise en place de la prothèse articulaire dans l'os fémoral pour permettre l'attache de la prothèse articulaire à celle-ci,
**caractérisé en ce que** la structure de montage d'os est sous forme d'une pièce moulée (132) qui est construite à partir des données anatomiques obtenues de la dite zone d'extrémité de l'os fémoral pour ainsi définir des structures de mise en place complémentaires (134) qui correspondent aux structures anatomiques (136) de la dite zone d'extrémité de l'os fémoral pour ainsi permettre l'attache sûre de la structure de montage d'os à la dite zone d'extrémité de l'os fémoral.

2. Guide de positionnement selon la revendication 1, dans lequel le support d'attache comprend une structure de liaison amovible (189) pour relier le support d'attache au dit guide de découpe d'os de manière amovible.

3. Guide de positionnement selon la revendication 1 ou la revendication 2, dans lequel le guide de positionnement comprend une paire de supports d'attache (154.1, 154.2).

4. Système de guide de découpe d'os fémoral (100) destiné à être utilisé dans une procédure d'opération de remplacement de genou pour découper au moins une surface de mise en place d'une prothèse articulaire dans une zone d'extrémité d'un os fémoral d'un patient humain, duquel os fémoral il faut enlever une partie d'os pour ainsi permettre d'attacher la prothèse articulaire de manière sûre à l'os fémoral dans une orientation prédéterminée qui est proche de la normalité anatomique de l'articulation du genou du patient, le système de guide de découpe d'os comprenant :
un ensemble de guide de découpe d'os (118) comprenant :
a) un guide de découpe d'os (124) ayant au moins une structure de guide de découpeur (150.1, 150.2, 150.3, 150.4, 150.5, 150.6) pour guider un découpeur (119) pour découper la dite surface de mis en place de la prothèse articulaire dans la dite zone d'extrémité de l'os fémoral ; et
b) des moyens de montage de guide susceptible d'être fermement attachés à l'os fémoral, lesquels comprennent des moyens de montage sur lesquels le guide de découpe d'os est monté de manière amovible pour monter le guide de découpe d'os par rapport à l'os fémoral de manière amovible quand les moyens de montage de guide sont attachés à celui-ci ; et
le système de guide de découpe d'os étant **caractérisé en ce que** le système de guide de découpe d'os comprend le guide de positionnement selon la revendication 1 pour monter l'ensemble de guide de découpe d'os sur l'os fémoral,
les moyens de montage de guide étant fermement attachés à l'os fémoral après le montage de l'ensemble de guide de découpe d'os sur le support d'attache, le support d'attache, les moyens de montage de guide et le guide de découpe d'os étant configurés de sorte qu'ils permettent une séparation du guide de découpe d'os du support d'attache et des moyens de montage de guide pour faciliter d'enlever le guide de positionnement et de remonter le guide de découpe d'os sur les moyens de montage de guide après avoir enlevé le guide de positionnement.

5. Système de guide de découpe d'os fémoral selon la revendication 4, dans lequel le guide de découpe d'os comprend des moyens de montage au support d'attache pour monter le guide de découpe d'os de manière amovible sur le support d'attache du guide de positionnement.

6. Système de guide de découpe d'os fémoral selon la revendication 4 ou la revendication 5, dans lequel le guide de découpe d'os comprend des moyens de montage complémentaires pour monter le guide de découpe d'os de manière amovible sur les moyens de montage des moyens de montage de guide.

7. Système de guide de découpe d'os fémoral selon la revendication 4, dans lequel le guide de positionnement comprend une paire de supports d'attache (154.1, 154.2) et dans lequel les moyens de montage au support d'attache du guide de découpe d'os sont sous forme d'une paire de structures complémentaires de montage aux supports d'attache (143.1, 143.2).

8. Système de guide de découpe d'os fémoral selon la revendication 6, dans lequel les moyens de montage complémentaires du guide de découpe d'os sont sous forme d'une paire de structures de montage complémentaires (147.1, 147.2), chaque structure de montage étant espacé des zones latérales opposées du guide de découpe d'os.

9. Système de guide de découpe d'os fémoral selon l'une quelconque des revendications 4 à 8, dans lequel les moyens de montage de guide de l'ensemble de guide de découpe d'os sont sous forme d'une paire de plaques latérales de montage (144.1, 144.2), chaque plaque latérale de montage comprenant des moyens de montage d'os pour monter la plaque de manière amovible à une région latérale opposée différente de l'os fémoral en usage.

10. Système de guide de découpe d'os fémoral selon l'une quelconque des revendications 4 à 9, dans lequel le guide de découpe d'os comprend un premier composant de guide de découpe (140) et un deuxième composant de guide de découpe (142), les composants de guide de découpe (140, 142) ayant des structures d'attache amovible complémentaires (147.1, 147.2) pour relier de manière amovible les composants de guide de découpe l'un à l'autre.
